# FASCICULE DE BREVET EUROPEEN

(11) **EP 0 311 482 B1**
(45) Date de publication et mention de la délivrance du brevet: **20.01.1993**
(21) Numéro de dépôt: 88402427.4
(22) Date de dépôt: 27.09.1988
(51) Int. Cl.: A61G 13/00

(54) **Table d'opération orthopédique pour membres, et notamment pour membres inférieurs**
Orthopädischer Operationstisch für Glieder, insbesondere für unterste Glieder
Orthopaedic operating table for limbs, especially for lower limbs

(30) Priorité: 05.10.1987 FR 8713708
(43) Date de publication de la demande: 12.04.1989
(73) Titulaire: ETABLISSEMENTS TASSERIT, F-89930 Gron (FR)
(72) Inventeur: Akcelrod, Patrick, F-89100 Sens (FR)
(74) Mandataire: Flavenot, Bernard

(56) Documents cités:
- EP-A- 0 240 430
- FR-A- 747 844
- FR-A- 1 319 436
- FR-A- 1 604 790
- US-A- 2 073 932
- US-A- 2 150 314
- US-A- 2 590 739
- US-A- 3 278 207
- US-A- 3 766 384

## Description

La présente invention concerne les tables qui permettent d'effectuer des opérations sur des patients atteints au niveau de leurs membres, par exemple de fractures qu'il faut absolument réduire dans les meilleures conditions, ces tables étant plus particulièrement adaptées aux interventions sur les membres inférieurs.

Il existe déjà des tables d'opérations orthopédiques, notamment sur les membres inférieurs, qui permettent aux chirurgiens d'opérer dans des conditions relativement aïsées.

De telles tables d'opération ont été décrites, notamment, dans le US-A-3 766 384 (sur lequel se fonde le préambule de la revendication 1) et le FR-A-787 844, et comportent généralement un pied assez large apte à être fixé sur le sol. Ce pied supporte en son sommet une embase sur laquelle sont généralement fixées, en déport sur l'un de ses côtés, une ou deux tablettes sur lesquelles sont aptes à reposer le tronc et la tête du patient. Sur le côté opposé à celui supportant les tablettes, l'embase supporte deux longerons articulés sensiblement autour de deux points situés à une distance relativement faible l'un de l'autre, ces longerons ayant une longueur généralement supérieure à celle des membres inférieurs d'un individu, pour porter en outre des éléments annexes qui permettent de fixer, par exemple, les pieds, et, éventuellement, des appuis intermédiaires pour maintenir la jambe au niveau du genou, sachant que ces appuis peuvent être déplacés le long des longerons sensiblement entre leurs deux extrémités.

Une telle table comporte aussi, positionné généralement au niveau de l'embase, un support dit "support pelvis" permettant de maintenir le bassin du patient, mais aussi de positionner l'entrejambe en un point de référence parfaitement déterminé et fixe par rapport à l'embase, pour permettre au chirurgien de bien positionner et aligner chaque membre du patient avant, notamment, de réduire les fractures le plus parfaitement possible, afin que le patient puisse retrouver l'usage du ou des membres fracturés remis dans leur configuration originelle.

Cependant, ces opérations se compliquent lorsqu'il est nécessaire, pour des opérations délicates, d'effectuer celles-ci avec l'aide d'une radiographie, et plus particulièrement d'une radiographie en continu. Pour effectuer des opérations dans de telles conditions, à la table sont associés une source d'émission d'un rayonnement permettant la radiographie, généralement des rayons X, et un récepteur adapté au rayonnement émis par la source, du genre scintillateur, apte à traiter et analyser l'image reçue et, éventuellement, l'afficher, par exemple, sur un écran vidéo. L'adjonction à une table d'opération de tels éléments augmente considérablement l'encombrement de l'ensemble de la structure et le chirurgien n'a alors pas touutes ses aises pour pouvoir opérer.

En effet, pendant ces opérations, il est nécessaire d'orienter le couple des éléments permettant la radiographie, c'est-à-dire la source de rayonnement et le récepteur, de façon qu'ils soient le mieux positionnés possible l'un par rapport à l'autre et par rapport au membre à analyser. Or, l'encastrement de la table ou sa surélévation en association avec une estrade constitue, avec les longerons et les appareillages associés, autant d'obstacles qui gênent et limitent le déplacement ou le débattement de des deux éléments.

La présente invention a pour but de pallier les inconvénients mentionnés ci-dessus et de réaliser une table d'opération orthopédique, notamment pour les membres inférieurs, qui permette au chirurgien d'opérer dans des conditions de facilité optimales, et qui constitue, avec tous ses éléments associés tels qu'une source de rayonnement radiographique et son récepteur, un ensemble qui soit au minimum de l'encombrement possible.

Plus précisément, la présente invention a pour objet une table d'opération orthopédique pour les membres d'un individu, notamment les membres inférieurs, comportant un pied apte à prendre position sur un sol, une embase montée au sommet dudit pied, au moins une tablette de support du tronc dudit individu, des moyens pour rattacher ladite tablette à ladite embase de façon que ladite tablette soit en déport par rapport à ladite embase, au moins un longeron, et des moyens pour monter ledit longeron pivotant sur ladite embase à proximité du point de rattachement dudit membre au tronc de l'individu, du côté de l'embase opposé à celui sur lequel sont disposés les moyens de rattachement de ladite tablette, la distance séparant ledit longeron, dans sa position sensiblement parallèle audit sol sur lequel est apte à prendre position ledit pied, par rapport à ce sol, étant supérieure à la hauteur de la source de rayonnement de radiographie ou du récepteur d'images radiographiques, caractérisée par le fait que ledit longeron comporte une partie centrale en forme de tube cylindrique de révolution creux réalisé en un matériau transparent à un rayonnement de radiographie émis par une source de rayonnement, que lesdits moyens pour monter ledit longeron pivotant sur ladite embase comportent une partie en saillie solidaire de ladite embase et une rotule dont la partie mâle est constituée par une partie sphérique convexe fixée sur ladite partie en saillie de l'embase par une patte et dont la partie femelle est constituée par un embout solidarisé à une première extrémité dudit longeron, ledit embout étant conformé, en son intérieur, en une partie complémentaire demi-sphérique concave formant une demi-coupelle comportant une ouverture par laquelle passe ladite patte de liaison, et qu'elle comporte en outre des moyens de blocage commandables de ladite rotule dans une position déterminée constitués par un patin de frein disposé à proximmité de la partie convexe et comportant sur sa face en regard de la partie convexe un matériau de friction, par une tige de transmission constituée par un tube creux cylindrique de révolution réalisé dans un matériau transparent au rayonnement de radiographie, une première extrémité de cette tige coopérant avec ledit patin et sa seconde extrémité émergeant à l'extrémité de la partie tubulaire dudit longeron, et par des moyens pour commander le déplacement de ladite tige de transmission par rapport à ladite partie tubulaire formant ledit longeron.

D'autres caractéristiques et avantages de la présente invention apparaîtront au cours de la description suivante donnée en regard du dessin annexé à titre illustrastif, mais nullement limitatif, dans lequel:
La figure 1 représente, vu en perspective, un mode de réalisation d'une table d'opération orthopédique selon l'invention.
La figure 2 représente, vu en coupe partielle schématisée, un mode de réalisation d'un longeron entrant dans la composition de la table selon la figure 1, cette coupe étant référencée II-II sur la figure 6.
La figure 3 est une coupe transversale du longeron selon la figure 2, référencée III-III sur cette figure 2.
La figure 4 représente une vue de côté schématisée d'une partie de la table d'opération orthopédique selon la figure 1, permettant d'expliciter sa structure et ses avantages.
La figure 5 est une représentation en coupe d'un élément constitutif de la table d'opération orthopédique selon la figure 1, en l'occurrence le support pelvis.
La figure 6 représente un autre mode de réalisation d'un élément structurel du longeron de la table selon la figure 1.

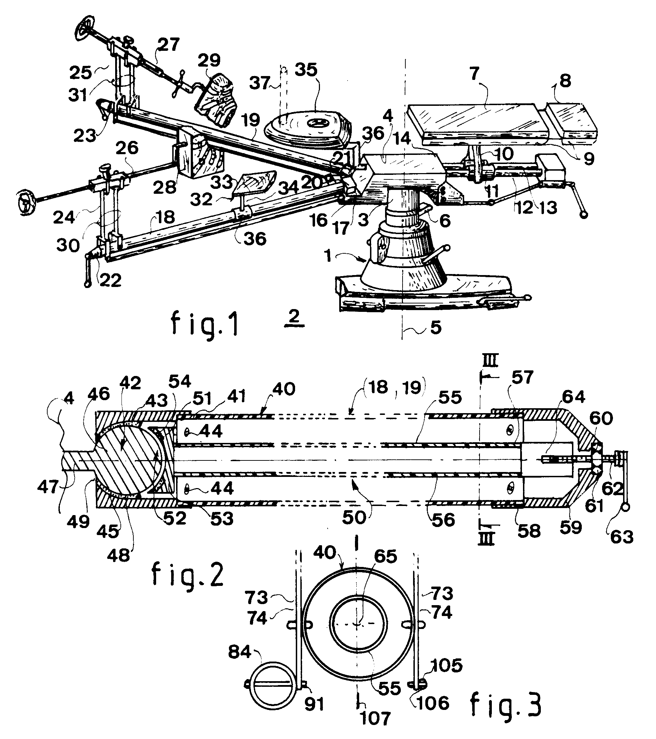

Il est bien évident que des éléments semblables représentés sur les figures 1 à 6 appartenant à un même mode de réalisation y sont désignés par les mêmes références, quelles que soient les figures sur lesquelles elles apparaissent.

La figure 1 représente, vu en perspective, un mode de réalisation d'une table d'opération orthopédique plus particulièrement adaptée pour des opérations sur les membres inférieurs d'un patient. Cette table comporte un pied 1 apte à prendre position de façon ferme sur un sol 2. Ce pied comporte à sa partie supérieure 3 une embase 4 de forme massique généralement montée pivotante autour d'un axe vertical 5, au moyen par exemple d'un palier avec roulement à billes ou analogue. De plus, ce palier peut être bloqué par tous moyens, notamment par un frein dont la commande 6 apparaît sur la figure. Ces moyens de blocage ne présentant pas de difficulté de mise en oeuvre pour un homme de l'art ne seront pas plus amplement décrits ici.

La table comporte en plus au moins une tablette, en l'occurrence deux 7, 8 montées généralement sur un même plateau 9 qui est lui-même monté sur des pattes 10. Ces pattes 10 sont généralement montées coulissantes par des manchons 11 sur deux glissières 12, 13. Celles-ci sont fixées sur une partie latérale 14 de l'embase 4. De même, ces manchons 11 pourront être bloqués dans une position choisie sur ces deux glissières. Ces moyens ne présentent pas, non plus, de difficulté de réalisation et ne seront pas plus amplement décrits. De cette façon, les deux tablettes 7 et 8 peuvent être positionnées par rapport à l'axe 5 défini ci-dessus, par translation le long des deux glissières 12 et 13, afin que le patient puisse reposer sur elles, par son tronc et la tête, ces deux tablettes ayant en plus des dimensions adaptées à celles de la partie du corps du patient qu'elles doivent supporter.

Sur le côté 16 de l'embase 4 opposé au côté 14 sur lequel sont montées les deux glissières 12 et 13, est prévue une partie en saillie 17 sur laquelle est monté rotatif au moins un longeron, et plus généralement deux, 18 et 19, qui servent respectivement de base de support aux deux membres inférieurs du patient. Chaque longeron 18, 19 est monté rotatif sur la partie en saillie 17 au moyen d'une rotule 20, 21 en association avec des moyens de bloquage 50. Sur chaque longeron, à son extrémité 22, 23 opposée à celle qui est montée rotative par l'intermédiaire de la rotule sur la partie en saillie de l'embase, sont montés des moyens de support et de maintien 24, 25 d'un pied du patient, ces moyens comportant essentiellement une tige support 26, 27 à l'extrémité de laquelle est montée une sorte de chaussure 28, 29, par exemple à lanières, dans laquelle peut être emprisonné le pied. Chaque tige 26, 27 est liée à l'extrémité 22, 23 du longeron 18, 19 par des pattes de fixation 30, 31 formant avantageusement un parallélogramme déformable, les extrémités de ces pattes étant respectivement montées pivotantes sur les longerons et sur les tiges supporte 26, 27. Ces derniers moyens sont bien connus en eux-mêmes et couramment utilisés dans les tables d'opérations orthopédiques selon l'art antérieur. Ils ne seront donc pas plus amplement décrits ici.

De plus, entre les deux extrémités de chaque longeron, est monté un appui de jambe 32 constitué, par exemple, par une portion de goulotte 33 solidaire d'une tige 34 elle-même solidaire d'un manchon 36 coulissant le long du longeron 18, 19 et pouvant, lui aussi, être bloqué par tous moyens dans une position déterminée entre les deux extrémités du longeron, afin que la goulotte 33 dans la rainure de laquelle est apte à être positionnée la jambe du patient puisse être située à l'endroit choisi par le chirurgien pour bien maintenir et soutenir le membre sur lequel il doit effectuer l'opération.

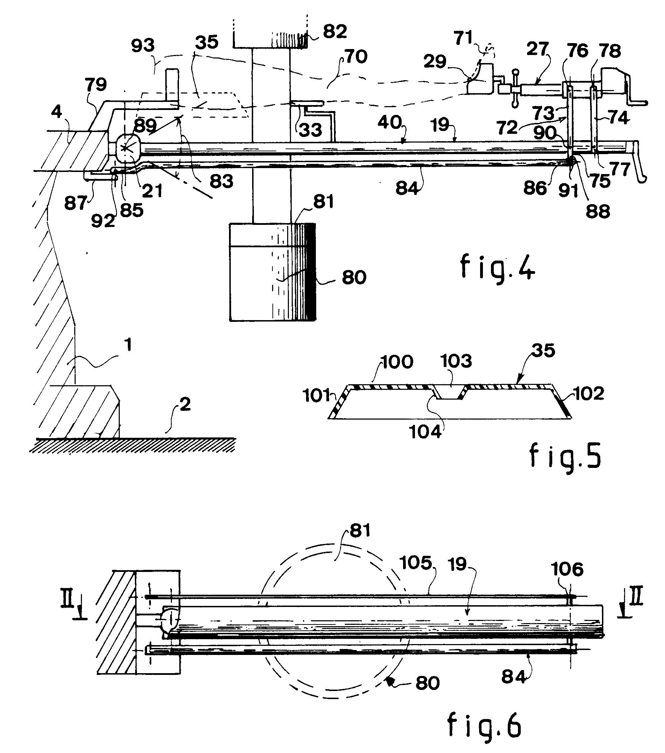

De plus, l'embase 4 comporte, en association avec son extrémité 16, un support pelvis 35 dont la forme avantageuse sera décrite ci-après, notamment en regard de la figure 5. Ce support pelvis est situé en déport de l'embase 4, du côté de l'espace contenant les deux longerons 18, 19 et de telle façon qu'il soit situé sensiblement au-dessus des deux longerons, les longerons et ce support pelvis étant donc du même côté du plan perpendiculaire au sol 2 et passant sensiblement par les deux points de rotation des deux rotules 20 et 21. Ce support pelvis 35 est réuni à l'embase 4 par une patte de fixation 36 qui permet d'obtenir son déport par rapport à cette embase et sa position définie ci-dessus. Eventuellement, ce support pelvis peut comporter une butée d'arrêt 37 qui permettra de définir la position de l'entre-jambe du patient dont le bassin repose sur le support pelvis 35 et le tronc sur les tablettes 7, 8.

Bien entendu, une telle table d'opération pourra comprendre d'autres éléments annexes connus en eux-mêmes et couramment utilisés pour de telles opérations.

Selon une caractéristique de la présente invention, les deux rotules 20, 21 de rotation associées aux deux longerons 18, 19 sont situées sur la partie en sailie 17 de façon qu'elles soient contenues dans un plan sensiblement horizontal très proche de celui contenant le support pelvis 35 et de celui passant par les deux tablettes 7, 8 sur lesquelles est apte à reposer le tronc du patient. Quant aux deux longerons, dans la partie située entre leurs deux extrémités, ils sont constitués par des poutres rectilignes avantageusement de forme telle que représenté sur la figure 2.

La figure 2 représente, à titre illustratif, l'un des deux longerons 18, 19 de la figure 1 suivant une coupe partielle longitudinale, sachant que ces deux longerons seront avantageusement de même structure. Un longeron 18, 19 est donc constitué d'une partie centrale en forme de tube cylindrique de révolution creux 40 réalisé en un matériau transparent au rayonnement utilisé pour effectuer la radiographie. Dans le cas de l'utilisation de rayons X, ce matériau est, par exemple, un composite de fibres de carbone tressées et assemblées de façon à former cette forme tubulaire cylindrique de révolution creuse.

La partie tubulaire 40 comporte, à une première extrémité 41, des moyens pour former une rotule 42. Ces moyens comportent un embout 43 solidarisé à l'extrémité 41 de la partie tubulaire, par exemple au moyen de goupilles 44, cet embout 43 étant conformé, en son intérieur, en une partie demi-sphérique concave 45 apte à coopérer avec une partie complémentaire sphérique convexe 46 qui, elle-même, a une partie fixée sur l'embase 4, par exemple par une patte 47. Entre ces deux parties sphériques femelle et mâle 45 et 46, peut être disposé un moyen 48 permettant de favoriser la friction de l'une sur l'autre. La partie femelle 45 forme une demi-coupelle comportant une ouverture 49 à travers laquelle passe la patte de liaison 47 de la partie mâle avec l'embase 4. Le tube 40 peut donc pivoter dans un angle solide d'une certaine valeur shématiquement représenté en 83 sur la figure 4, pour permettre à la jambe d'être positionnée par rapport au tronc dans le même angle solide, si cela peut faciliter l'intervention du chirurgien.

Bien entendu, quand le longeron aura pris la position désirée par le chirurgien, il pourra être bloqué dans cette position par les moyens de blocage 50. Dans une forme avantageuse de réalisation, ces moyens de blocage 50 comprennent un patin de frein 51 disposé à proximité de la tête 52 de la partie sphérique mâle 46, ce patin de frein pouvant être constitué par une partie massique 53 comportant, sur sa face 54 en regard de la tête 52 de la partie sphérique mâle, un matériau de frein bien connu favorisant la friction, notamment comme celui qui est utilisé dans les garnitures de freins pour les véhicules automobiles.

Le patin 51 est monté à l'une des extrémités d'une tige 55 constituée par un tube creux 56 cylindrique de révolution réalisé, lui aussi, dans un matériau transparent au rayonnement de radiographie. L'autre extrémité 57 de ce tube intérieur 56 émerge à l'extrémité 58 de la partie tubulaire 40 du longeron opposée à l'extrémité 41 de façon que l'on puisse agir sur elle pour déplacer le patin et le faire venir, soit au contact de la tête 52 de la partie sphérique mâle 46, soit en une position éloignée.

Les moyens pour commander le déplacement de cette tige 55 sont constitués par un deuxième embout 59 solidarisé avec la partie tubulaire 40 à son extrémité 58 et comportant une ouverture 60 dans laquelle est fixé, par exemple, un pas de vis 61 dans lequel est disposée une vis 62 apte à être commandée par une manivelle 63. Cette vis 62 coopère avec la partie extrême 64 de la tige 55 de façon que, lorsque l'on tourne la manivelle 63, l'extrémité de la vis 62 éloigne ou rapproche l'extrémité de la tige 55 de ce pas de vis 61, et le patin se rapproche ou s'éloigne de la tête 52 de la partie sphérique mâle. On pourra ainsi bloquer la rotule par une forte pression du patin favorisée par la nature du matériau 54, ou la libérer pour changer et ajuster l'orientation des longerons 18, 19. En fait, le blocage du longeron est réalisé par une traction sur sa partie tubulaire 40 par rapport au tube intérieur 55, de façon à pincer fortement la partie sphérique convexe 46 entre la portion sphérique concave 45 et le patin 53.

Les moyens permettant le déplacement en translation de la tige de frein 55 n'ont été donnés qu'à titre d'exemple de mode de réalisation possible, mais il est bien évident qu'il pourra être utilisé d'autres moyens pour créer ce déplacement.

En se reportant maintenant plus particulièrement à la figure 3 qui représente une coupe transversale d'un longeron référencée III-III sur la figure 2, cette figure illustre le fait que les deux parties tubulaires 40 et 55 cylindriques de révolution sont centrées sur un même axe 65. Bien que, comme mentionné précédemment, le matériau dans lequel elles sont réalisées soit un matériau transparent au rayonnement radiographique, les rayons émis par la source de rayonnement sont donc amenés à traverser quatre épaisseurs de paroi avant de radiographier le membre blessé et d'être reçus par le récepteur 82. Mais la configuration du longeron permet aux parois d'être constamment perpendiculaires au rayonnement et leur épaisseur est très faible. L'absorption des rayons émis est donc pratiquement nulle. Par ailleurs, il est à remarquer que les longerons, bien que réalisés dans un matériau de très faible épaisseur, présentent malgré tout une bonne rigidité apportée par leur forme cylindrique de révolution.

La source de rayonnement X telle que représentée sur la figure 4 pourra donc être placée en dessous du longeron par rapport à la jambe du patient, sans que ce longeron puisse nuire à la qualité de la vision radiographique. Il en sera de même si l'agencement de la salle d'opération fait préférer de disposer le récepteur d'images radiographiques dans l'espace entre les longerons et le sol, la source de rayonnement étant alors située au-dessus de la jambe malade. L'une ou l'autre configuration permet, avec les caractéristiques mentionnées ci-avant, d'obtenir un ensemble des éléments constituant cette table d'opération présentant un minimun d'encombrement. Le chirurgien pourra donc travailler dans des conditions de facilité optimales, sans subir la moindre gêne de la part de ces éléments.

La figure 4 représente un schéma d'une partie de la table d'opération selon la figure 1, dans une vue de côté, plus précisément la partie comportant le longeron 19. La jambe 70 du patient peut être positionnée, par rapport à ce longeron, de façon que son pied 71 soit disposé dans le moyen de chaussure 29 monté sur la tige 27. Cette tige 27 est montée parallèlement à la partie cylindrique 40 au moyen d'un parallélogramme déformable 72 constitué d'au moins deux tiges 73, 74 (en fait généralement quatre) montées pivotantes à leurs deux extrémités 75,76 - 77,78, respectivement sur le longeron 19, et plus particulièrement sur sa partie 40, et sur la tige 27. Ces deux tiges 73, 74 étant parallèles et ayant une même longueur prise entre leurs axes de rotation respectivement sur ces deux éléments, lorsque l'on déplace angulairement l'une d'elles par rapport aux deux éléments, longeron 40 et tige 27, elles restent toujours parallèles.

Selon une caractéristique importante de l'invention, l'axe de rotation de chaque longeron est le plus proche possible du siège pelvis 35, c'est-à-dire du point du corps du patient au niveau duquel la jambe est reliée au tronc. De cette façon, lorsque le chirurgien est amené à changer l'orientation de la jambe dans les limites d'une amplitude relativement restreinte, il lui suffit d'agir sur le longeron, sans avoir à changer le réglage des pattes 73, 74 et du support 29 du pied, car, dans ce cas, l'appareillage n'exerce pas sur la jambe une traction importante nuisible et la déformation du parallélogramme 72 est suffisante pour conserver la jambe dans un bon état d'alignement.

Par ailleurs, le plan des longerons lorsqu'ils sont en position horizontale est sensiblement confondu avec celui de l'embase et très proche de celui des tablettes 7, 8 et du support pelvis 35 et l'espace compris entre les longerons et le sol 2 sur lequel repose la table par son pied 1 est très important. Ceci permet d'y positionner, par exemple, une source de rayons X 80 de façon que sa surface émettrice 81 soit très proche de la jambe 70. Comme mentionné précédemment, bien qu'un longeron soit interposé entre la source de rayonnement et la jambe, du fait de la faible épaisseur des parois et de la nature du matériau le constituant, il ne constitue pas un obstacle à la réalisation d'une image de bonne qualité sur le récepteur 82 positionné en regard de la surface d'émission de la source.

Comme décrit ci-dessus, le parallélogramme déformable 72 permet de maintenir constamment là tige 27 parallèle au longeron. Cependant, comme évoqué précédemment, le chirurgien peut être amené à changer l'orientation de la jambe par rapport au plan de l'embase 4. Pour effectuer cette manoeuvre, il peut débloquer le patin de frein 53 comme explicité en regard de la figure 2, par exemple au moyen de la manivelle 63, puis orienter comme il le faut le longeron 19 dans l'angle solide schématisé en 83. Le parallélisme de la tige 27 par rapport au longeron est maintenu grâce au parallélogramme déformable 72 décrit ci-avant. Cependant, si le changement d'orientation de la jambe est important, il s'exerce sur elle des efforts de traction ou de tassement pouvant être dangereux. Pour éviter la création de tels efforts, la table d'opération orthopédique comporte en outre une biellette de commande 84 montée pivotante à ses deux extrémités 85 et 86, respectivement sur une partie 87 de l'embase 4 et sur une partie en saillie 88 de la patte 73 du parallélogramme 72, de façon que les points de rotation 89 de la rotule du longeron, 90 de la patte 73 sur le longeron 19, 91 de la biellette 84 sur la saillie 88 de la patte 73 et 92 de la biellette 84 sur l'embase 4 soient sensiblement situés aux sommets d'un trapèze.

En effet comme mentionné précédemment, le point de rotation 89 de la rotule ne peut pas être parfaitement confondu avec le point de rotation de la jambe 70 par rapport au bassin 93 qui se trouve positionné sur le siège pelvis 35. Aussi, lorsqu'il est nécessaire de faire pivoter ce longeron 19 par rapport à l'embase 4, pour compenser les variations de distance qui pourraient exister entre le bassin 93 et le moyen de maintien du pied 71, l'axe de rotation 92 de la biellette 84 sur l'embase 4 est légèrement décalé vers l'arrière, c'est-à-dire vers le pied 1, de façon que la distance prise entre les deux axes 91 et 92 soient supérieure à celle prise entre les deux axes 89 et 90. Ainsi, lorsque le longeron subit une rotation ascendante, le moyen de maintien du pied 71 reste à distance sensiblement constante du support pelvis. En effet, la biellette 84 tire alors sur la patte 73 pour faire subir à son extrémité 76 une légère rotation dextrorsum qui a tendance à l'éloigner du support pelvis 35, de façon à compenser le rapprochement dû à la rotation du longeron autour de la rotule.

De même, quand le longeron 19 subit une rotation dextrorsum, c'est-à-dire vers le sol, la patte 73 subit une rotation senestrorsum afin de compenser l'éloignement relatif du moyen de support du pied et du support pelvis, de façon que leur distance soit aussi, dans ce cas, maintenue sensiblement constante.

Selon une autre caractéristique de cette invention, le support pelvis 35 est situé entre les deux plans verticaux passant respectivement par le point de rotation 89 du longeron 19 sur l'embase 4 et par le pied 71 du patient, de façon que ce support pelvis puisse être introduit dans le champ de rayonnement de la source de rayons X. Ce support 35 est, lui aussi, réalisé en un matériau transparent, notamment aux rayons X, par exemple un composite de fibres de carbone. Le bassin du patient peut reposer sur le siège pelvis et y être alors radiographié en continu. Cette caractéristique permet donc d'effectuer l'opération de la hanche ou de réduire des fractures du bassin avec contrôle continu par radiographie.

Pour éviter que ce siège pelvis, qui supporte un poids relativement important, ne se déforme, il est réalisé de façon à être relativement rigide. Comme illustré sur la figure 5, il peut être constitué d'une paroi mince 100 conformée pour recevoir le bassin du patient, mais renforcée latéralement par des flancs 101, 102, et même percée sensiblement en son centre d'une ouverture 103 elle-même bordée de flancs 104, ces flancs 101, 102 et 104 ayant par rapport à la perpendiculaire à la paroi 100 des directions obliques afin que l'épaisseur du siège, prise suivant une direction parallèle à cette perpendiculaire, reste en tous points faible.

Pour commander la déformation du parallélogramme 72 comme décrit ci-avant, il est utilisé une biellette 84 telle que décrite ci-dessus disposée parallèlement au longeron 18, 19. Mais, pour qu'elle ne gêne pas la transmission du rayonnement radiographique entre la source et la jambe, elle est située latéralement par rapport à la partie tubulaire 40, de façon que cette biellette et le longeron soient situés de part et d'autre d'un plan perpendiculaire au sol 2 et tangent à au moins l'un de ces deux éléments. En particulier, ce plan peut être celui contenant la patte 73 et parallèle au longeron 19, et la biellette 84 et la partie tubulaire 40 sont donc situées de part et d'autre de cette patte 73.

Comme la biellette 84 constitue un moyen de transmission d'efforts à l'extrémité 23 de la partie tubulaire 40 par l'intermédiaire de la partie en saillie 88 solidaire de la patte 73, elle a tendance à se déformer en arc de cercle et à venir se plaquer sensiblement en son milieu contre la paroi de la partie tubulaire 40, ce qui contribuerait alors à augmenter l'épaisseur de matière à traverser dans la direction verticale pour le rayonnement de radiographie et plus particulièrement de rayons X.

Pour éviter cet inconvénient, à chaque biellette 84 est associé un tirant latéral 105. Or, pour un meilleur équilibre de la tige support 27, et donc du moyen de maintien du pied 29, chaque patte 73, 74 est avantageusement constituée de deux pattes symétriques par rapport au plan 107 longitudinal passant par l'axe central 65 du longeron. Dans ce cas, la biellette est montée en rotation sur l'une des deux pattes 73 et le tirant est monté, lui, sur la deuxième, en prenant appui en un point 106 de cette deuxième patte 73 symétrique du point de rotation 91 défini ci-dessus par rapport au plan vertical 107. L'autre extrémité du tirant est montée rotative sur la partie 87 de l'embase 4 en un point symétrique du point de rotation 92 de la biellette par rapport au même plan 107. De cette façon, les quatre points de fixation de la biellette et du tirant forment un rectangle qui va pouvoir se déformer, essentiellement élastiquement, sous l'effet de la contrainte exercée par le déplacement du longeron, ce qui empêchera la biellette de se déformer comme décrit ci-avant.

La table décrite possède incontestablement un nombre d'avantages de par sa structure et la nature de certains de ses éléments constitutifs, tels que les longerons et le support pelvis. Elle permet notamment au chirurgien de pouvoir opérer et/ou réduire les fractures des membres inférieurs et du bassin en bénéficiant d'un contrôle continu par radiographie, les longerons et le support pelvis étant réalisés dans un matériau transparent au rayonnement de radiographie. Les formes structurelles de ses éléments principaux leur donnent une grande rigidité et une très bonne solidité, malgré leur faible épaisseur. De plus, comme la source de rayonnement ou le récepteur d'images radiographiques est apte à être disposé entre le plan des longerons et le sol et comme les plans des différents éléments, embase, tablettes, support pelvis et longerons en position horizontale, sont très proches les uns des autres, encombrement total de cette table est réduit au minimum et le travail du chirurgien en est grandement facilité. Il peut en effet avoir très facilement accès à tous les moyens fonctionnels qui lui sont utiles pour opérer avec contrôle continu par radiographie. Les opérations peuvent être encore plus rapides et plus sûres, ce qui est le but principal recherché pour le bien-être des patients.

## Revendications

1. Table d'opération orthopédique pour les membres d'un individu, notamment les membres inférieurs, comportant :
un pied (1) apte à prendre position sur un sol (2),
une embase (4) montée au sommet dudit pied,
au moins une tablette de support (7,8) du tronc dudit individu,
des moyens (10-13) pour rattacher ladite tablette à ladite embase de façon que ladite tablette soit en déport par rapport à ladite embase,
au moins un longeron (18,19), et
des moyens pour monter ledit longeron pivotant sur ladite embase à proximité du point de rattachement dudit membre au tronc de l'individu, du côté de l'embase opposé à celui sur lequel sont disposés les moyens de rattachement de ladite tablette, la distance séparant ledit longeron, dans sa position sensiblement parallèle audit sol sur lequel est apte à prendre position ledit pied, par rapport à ce sol, étant supérieure à la hauteur de la source de rayonnement de radiographie ou du récepteur d'images radiographiques,
caractérisée par le fait
que ledit longeron comporte une partie centrale en forme de tube cylindrique de révolution creux (40) réalisé en un matériau transparent à un rayonnement de radiographie émis par une source de rayonnement (80,81),
que lesdits moyens pour monter ledit longeron pivotant sur ladite embase comportent une partie en saillie (17) solidaire de ladite embase (4) et une rotule (20,21,42) dont la partie mâle (46) est constituée par une partie sphérique convexe (46) fixée sur ladite partie en saillie de l'embase (4) par une patte (47) et dont la partie femelle (45) est constituée par un embout (43) solidarisé à une première extrémité (41) dudit longeron, ledit embout étant conformé, en son intérieur, en une partie complémentaire demi-sphérique concave (45), formant une demi-coupelle comportant une ouverture (49) par laquelle passe ladite patte de liaison (47), et
qu'elle comporte en outre des moyens de blocage commandables (50) de ladite rotule dans une position déterminée constitués par un patin de frein (51) disposé à proximité de la partie convexe (52) et comportant sur sa face (54) en regard de la partie convexe (52) un matériau de friction, par une tige de transmission (55) constituée par un tube creux (56) cylindrique de révolution réalisé dans un matériau transparent au rayonnement de radiographie, une première extrémité de cette tige coopérant avec ledit patin et sa seconde extrémité (57) émergeant à l'extrémité (58) de la partie tubulaire (40) dudit longeron, et par des moyens pour commander le déplacement de ladite tige (55) de transmission par rapport à ladite partie tubulaire (40) formant ledit longeron.

2. Table selon la revendication 1, caractérisée par le fait que la rotule (42) comporte, entre les deux parties sphériques concave et convexe (45,46), des moyens (48) pour favoriser la friction de l'une sur l'autre.

3. Table selon l'une des revendications 1 et 2, caractérisée par le fait que lesdits moyens pour commander le déplacement de ladite tige (55) sont constitués par un deuxième embout (59) solidarisé avec la partie tubulaire (40) à son extrémité (58) opposée à celle coopérant avec ladite rotule, cedit deuxième embout (59) comportant une ouverture (60) dans laquelle est fixé un pas de vis (61) dans lequel est disposée une vis (62) solidaire de ladite tige de transmission (55), et une manivelle (63) de commande en rotation de ladite vis (62).

4. Table selon l'une des revendications 1 à 3, caractérisée par le fait
que ledit longeron comporte, à sa seconde extrémité (22,23), des moyens de support et de maintien (24,25) d'un pied du patient comportant une tige support (26,27), une chaussure (28,29) dans laquelle peut être emprisonné le pied du patient et solidaire d'une extrémité de ladite tige, un parallélogramme déformable (72) liant ladite tige audit longeron, ledit parallélogramme étant constitué par des pattes de fixation (30,31) les extrémités de ces pattes étant respectivement montées pivotantes sur ladite seconde extrémité dudit longeron et sur la tige support, et
qu'elle comporte en outre une biellette de commande (84) dudit parallélogramme déformable montée pivotante à ses deux extrémités (85,86), respectivement sur une partie (87) de ladite embase (4) et sur une partie en saillie (88) d'une patte (73) formant ledit parallélogramme (72), de façon que les points de rotation (89,90,91,92) de ladite rotule (42), de la patte (73) sur le longeron (19), de la biellette (84) sur la patte (73) et de la biellette (84) sur l'embase (4) soient situés aux sommets d'un trapèze, la longueur de ladite biellette (84) prise entre ses deux points de rotation (91,92) étant supérieure à la longueur dudit longeron prise entre ses deux dits points de rotation (89,90), ladite biellette (84) et ladite partie tubulaire (40) forment le longeron étant situées de part et d'autre d'un plan sensiblement perpendiculaire audit sol sur lequel est apte à être positionné ledit pied (1).

5. Table selon la revendication 4, caractérisée par le fait qu'elle comporte en outre un tirant (105) relié à ladite embase et audit longeron, la longueur dudit tirant prise entre ses deux points de liaison avec ladite embase et ledit longeron étant sensiblement égale à celle de ladite biellette (84), ledit tirant étant situé sensiblement dans un plan symétrique de celui passant par l'axe longitudinal de ladite biellette (84) et perpendiculaire audit sol, par rapport à un plan (107) perpendiculaire audit sol passant par l'axe longitudinal dudit longeron (18,19).

6. Table selon l'une des revendications 1 à 5, caractérisée par le fait qu'elle comporte en outre un support pelvis (35) situé dans le demi-espace délimité par le plan passant par le point de rotation dudit longeron et sensiblement perpendiculaire au sol sur lequel est apte à se positionner ledit pied, et comprenant ledit longeron (18,19), et constitué d'une paroi mince (100) conformée pour recevoir le bassin d'un patient, ladite paroi mince étant renforcée latéralement par des flancs (101,102,104) à épaisseur faible ayant, par rapport à la perpendiculaire à ladite paroi mince, une direction oblique, ledit support pelvis (35) étant réalisé en un matériau transparent au rayonnement de radiographie.

7. Table selon l'une des revendications 1 à 6, caractérisée par le fait que ladite source de rayonnement est une source de rayons "X", et que ledit matériau transparent audit rayonnement est un composite de fibres de carbone tressées.

## Claims

1. An orthopedic operating table for the limbs of a person, in particular the lower limbs, the table comprising :
a stand (1) suitable for being positioned on the ground (2),
a base (4) mounted on the top of said stand,
at least one flap (7, 8) for supporting the trunk of said person,
support means (10-13) for attaching said flap to said base so that said flap is offset relative to said base,
at least one beam (18, 19),
means for mounting said beam pivotally to said base in the proximity of the point where said limb joins the trunk of the person, and on the opposite side of the base to the side on which the support means for said flap are disposed, with the distance between said beam, when in its position substantially parallel to said ground on which said stand is suitable for being positioned, and said ground, being greater than the height of the source of radiographic radiation or of the radiographic image receiver,
characterized by the fact that:
said beam comprises a central portion in the form of a hollow circularly tube (10) made of a material which is transparent to radiographic radiation emitted by a radiation source (80, 81),
said means for mounting said beam pivotally to said base comprise a projecting portion (17) fixed to said base (4) and a ball-and-socket joint (20, 21, 42) whose male portion (40) is constituted by a convex spherical portion (46) fixed to said projecting portion of the base (4) by a lug (47) and whose female portion (45) is constituted by an end piece (43) fixed to a first end (41) of said beam, the inside of said end piece being shaped to include a complementary concave hemispherical portion (45) forming a half-cup including an opening (49) through which said connection lug (47) passes, and that
it further includes controllable locking means (50) for locking said ball-and-socket joint in a determined position constituted by a brake shoe (51) disposed in the proximity of the convex portion (52) and including a friction material on its face (54) facing the convex portion (52), a transmission rod (55) constituted by a circulary cylindrical hollow tube (56) made of a material transparent to the radiographic radiation, with a first end of said rod cooperating with said shoe and with its second end (57) emerging from the end (58) of the tubular portion (40) of said beam, and means for controlling the displacement of said transmission rod (55) relative to said tubular portion (40) forming said beam.

2. Table according to claim 1, characterized by the fact that the ball-and-socket joint (42) includes means (48) between its concave and convex spherical portions (45, 46) for increasing the friction therebetween.

3. Table according to any one of claims 1 and 2, characterized by the fact that said means for controlling displacement of said rod (55) are constituted by a second end piece (59) fixed to the end (58) of the tubular portion (40) opposite from the end cooperating with said ball-and-socket joint, said second end piece (59) comprising an opening (60) in which a screw thread (61) is fixed receiving a screw (62) associated with said rod (55), and a handle (63) for controlling the rotation of said screw (62).

4. Table according to any one of claims 1 to 3, characterized by the fact that:
said beam comprises, at its second end (22, 23), means (24, 25) for supporting and holding a foot of the patient, said means comprising a support rod (26, 27), a boot (28, 29) fixed to one end of said rod and suitable for imprisoning a foot of the patient, a deformable parallelogram (72) connecting said rod to said beam, said parallelogram being constituted by fixing lugs (30, 31), with the ends of said lugs being pivotally mounted respectively on said second end of said beam and on said support rod, and that
it further comprises a control link (84) for controlling said deformable parallelogram, said control link being pivotally mounted at its two ends (85, 86), respectively on a portion (87) of said base (4) and on a projecting portion (88) of a lug (73) forming said parallelogram (72), such that the points of rotation (80, 90, 91, 92) of said ball-and-socket joint (42), of the lug (73) on the beam (19), of the link (84) on the lug (73), and of the link (84) on the base (4) lie on the vertices of a trapezium, with the length of said link (84) between its two points of rotation (91, 92) being greater than the length of said beam between its two points of rotation (89, 90), said link (84) and the tubular portion (40) constituting the beam being situated on either side of a plane substantially perpendicular to said ground on which said stand (1) is suitable for being positioned.

5. Table according to claim 4, characterized by the fact that it further includes a draw-bar (105) connected to said base and to said beam, with a length of said draw-bar between its two connection points with said base and said beam being substantially equal to the length of said link (84), said draw-bar being situated substantially in a plane symmetrical about a plane (107) perpendicular to said ground and passing through the longitudinal axis of said beam (18, 19), to the plane passing through the longitudinal axis of said link (84) and perpendicularly to said ground.

6. Table according to any one of claims 1 to 5, characterized by the fact that it further includes a pelvis support (35) situated in the half-space delimited by the plane passing through the point of rotation of said beam and substantially perpendicularly to the ground on which said stand is suitable for being positioned, and including said beam (18, 19), said pelvis support being constituted by a thin wall (100) shaped to receive the pelvis of a patient, said thin wall being laterally reinforced by thin sides (101, 102, 104) extending obliquely relative to the normal to said thin wall, said pelvis support (35) being made of a material which is transparent to radiographic radiation.

7. Table according to any one of claims 1 to 6, characterized by the fact that said source of radiation is an X-ray source, and said material which is transparent to said radiation is a braided carbon fiber composite.

## Patentansprüche

1. Orthopädischer Operationstisch für die Glieder einer Person, insbesondere der unteren Glieder, umfassend:
einen Fuß (1), der auf einem Boden (2) positionierbar ist,
eine an der Oberseite des Fußes montierte Basis (4),
mindestens eine Stütztafel (7,8) für den Rumpf der Person,
Mittel (10 bis 13) zum Befestigen der Tafel an der Basis derart, daß die Tafel gegenüber der Basis auskragt,
mindestens einen Längsträger (18,19), und
Mittel zum Montieren der Längsträger, schwenkbeweglich an der Basis nahe dem Verbindungspunkt des Gliedes mit dem Rumpf der Person auf der Seite der Basis, die abgekehrt ist derjenigen, an der die Mittel zum Befestigen der Tafel angeordnet sind, wobei der Abstand zwischen dem Längsträger, in seiner Position im wesentlichen parallel zu dem Boden, auf dem der Fuß positionierbar ist, und diesem Boden größer ist als die Höhe der Radiographiestrahlungsquelle oder des Radiographiebildempfängers, dadurch gekennzeichnet,
daß der Längsträger einen zentralen Abschnitt in Form eines Hohlzylinders (40) umfaßt, gefertigt aus einem für die von einer Strahlungsquelle (80,81) ausgehende Radiographiestrahlung transparenten Material,
daß die Mittel zum schwenkbeweglichen Montieren des Längsträgers an der Basis eine vorspringende Partie (17) umfassen, die mit der Basis (4) verbunden ist, und ein Kugelgelenk (20,21,42), dessen Kugelpartie (46) von einer konvexen, sphärischen Partie (46) gebildet ist, die an der vorspringenden Partie der Basis (4) mittels einer Pratze (47) befestigt ist, und dessen Sitzpartie (45) von einem Ansatzstück (43) gebildet ist, das verbunden ist mit einem ersten Ende (41) des Längsträgers, welches Ansatzstück in seinem Inneren mit einer halbsphärischen, konkaven, komplementären Partie (45) ausgebildet ist, das eine Halbkuppel mit einer Öffnung (49) bildet, durch welche die Verbindungspratze (47) ragt, und
daß er ferner steuerbare Blockiermittel (50) für das Kugelgelenk in einer bestimmten Position umfaßt, gebildet von einem Bremsschuh (51), der am Ende der konvexen Partie (52) angeordnet ist, und auf seiner der konvexen Partie (52) zugekehrten Seite (54) ein Reibungsmaterial umfaßt, von einer Übertragungsstange (55), gebildet von einem zylindrischen, hohlen Rohr (56), gefertigt aus einem für die Radiographiestrahlung transparenten Material, wobei ein erstes Ende dieser Stange mit dem Schuh zusammenwirkt und sein zweites Ende (57) aus dem Ende (58) der rohrförmigen Partie (40) des Längsträgers austritt, sowie von Mitteln zum Steuern der Verlagerung der Übertragungsstange (55) relativ zu der rohrförmigen Partie (40), welche den Längsträger bildet.

2. Tisch nach Anspruch 1, dadurch gekennzeichnet, daß das Kugelgelenk (42) zwischen den beiden konkaven und konvexen, sphärischen Partien (45,46) Mittel (48) umfaßt zum Begünstigen der Friktion der einen auf der anderen.

3. Tisch nach einem der Ansprüche 1 und 2, dadurch gekennzeichnet, daß die Mittel zum Steuern der Verlagerung der Stange (55) von einem zweiten Ansatzstück (59) gebildet werden, verbunden mit der rohrförmigen Partie (40) an ihrem dem mit dem Kugelgelenk zusammenwirkenden abgekehrten Ende (58), welches zweite Ansatzstück (59) eine Öffnung (60) aufweist, in der eine Spindelmutter (61) befestigt ist, in der eine Spindel (62), welche mit der Übertragungsstange (55) verbunden ist, angeordnet ist, und von einer Steuerkurbel (63) für die Drehung der Spindel (62).

4. Tisch nach einem der Ansprüche 1 bis 3, dadurch gekennzeichnet,
daß der Längsträger an seinem zweiten Ende (22,23) Stütz- und Haltemittel (24,25) für einen Fuß des Patienten aufweist, umfassend eine Stützstange (26,27), einen Schuh (28,29), in dem der Fuß des Patienten festlegbar ist und der mit einem Ende der genannten Stange verbunden ist, ein deformierbares Parallelogramm (72), das die genannte Stange mit dem Längsträger verbindet, welches Parallelogramm von Befestigungspratzen (30,31) gebildet wird, wobei die Enden dieser Pratzen schwenkbeweglich an dem zweiten Ende des Längsträgers beziehungsweise an der Stützstange montiert sind, und
daß er ferner einen Steuerlenker (84) für das deformierbare Parallelogramm umfaßt, der schwenkbeweglich an seinen beiden Enden (85,86) mit einer Partie (87) der Basis (4) beziehungsweise einem Vorsprung (88) einer Pratze (73) verbunden ist, welche das Parallelogramm (72) bildet, derart, daß die Drehpunkte (89,90,91,92) des Kugelgelenks (42) der Pratze (73) auf dem Längsträger (19), des Lenkers (84) auf der Pratze (73) und des Lenkers (84) an der Basis (4) auf den Spitzen eines Trapezes positioniert sind, wobei die Länge des Lenkers (84) zwischen seinen beiden Drehpunkten (91,92) größer ist als die Länge des Längsträgers zwischen seinen beiden Drehpunkten (89,90), wobei der Lenker (84) und die rohrförmige Partie (40), welche den Längsträger bildet, sich beidseits einer Ebene befinden, die im wesentlichen senkrecht zum Boden verläuft, auf dem der Fuß (1) positionierbar ist.

5. Tisch nach Anspruch 4, dadurch gekennzeichnet, daß er ferner einen Zuganker (105) umfaßt, der mit der Basis und mit dem Längsträger verbunden ist, wobei die Länge dieses Zugankers, gemessen zwischen seinen beiden Verbindungspunkten mit der Basis beziehungsweise dem Längsträger, im wesentlichen gleich ist derjenigen des Lenkers (84), welcher Zuganker sich im wesentlichen in einer Ebene symmetrisch zu derjenigen befindet, die durch die Längsachse des Lenkers (84) verläuft und senkrecht zum Boden, relativ zu einer Ebene (107), senkrecht zu dem Boden und durch die Längsachse des Längsträgers (18,19) verlaufend.

6. Tisch nach einem der Ansprüche 1 bis 5, dadurch gekennzeichnet, daß er ferner eine Hüftstütze (35) umfaßt, angeordnet in dem Halbraum, begrenzt von der durch den Drehpunkt des Längsträgers verlaufenden Ebene, die im wesentlichen senkrecht zum Boden ist, auf dem der Fuß positionierbar ist, und den Längsträger (18,19) umfassend, und gebildet von einer dünnen Wandung (100), angepaßt zum Aufnehmen des Beckens eines Patienten, welche dünne Wandung seitlich durch Flanken (101,102,104) verstärkt ist mit einer geringen Dicke, welche relativ zu der Senkrechten auf die dünne Wandung eine schräge Richtung haben, welche Hüftstütze (35) aus einem für die Radiographiestrahlung transparenten Material gefertigt ist.

7. Tisch nach einem der Ansprüche 1 bis 6, dadurch gekennzeichnet, daß die Strahlungsquelle eine Röntgenstrahlenquelle ist, und daß das für die Strahlung transparente Material ein Kompositmaterial mit Kohlenstoffaserschnüren ist.
